# EUROPÄISCHE PATENTSCHRIFT

(11) **EP 0 394 838 B1**
(45) Veröffentlichungstag und Bekanntmachung des Hinweises auf die Patenterteilung: **05.07.1995**
(21) Anmeldenummer: 90107431.0
(22) Anmeldetag: 19.04.1990
(51) Int. Cl.: C07D 301/19, C07D 303/38, C07D 303/40, C07C 69/732

(54) **Verfahren zur Herstellung von 3,4-Epoxialkan- und/oder 4-Hydroxi-2-alkensäuren und deren Ester**
Process for the preparation of 3,4-epoxyalkane- and/or 4-hydroxy-2-alkene carbonic acids and their esters
Procédé pour la préparation des acides 3,4-époxy-alcanoiques et/ou 4-hydroxy-2-alcéniques et leurs esters

(30) Priorität: 22.04.1989 DE 3913331
(43) Veröffentlichungstag der Anmeldung: 31.10.1990
(73) Patentinhaber: BASF Aktiengesellschaft, 67063 Ludwigshafen (DE)
(72) Erfinder: Rieber, Norbert, Dr., D-6800 Mannheim (DE)

(56) Entgegenhaltungen:
- EP-A- 0 097 551
- EP-A- 0 208 272
- EP-A- 0 232 752
- BE-A- 786 635
- PENNY A CHALONER: "Handbook of coordination catalysis in organic chemistry"
- & Co Ltd., London
- Ind.& Eng. Chem.5 (1966), S. 166-73

## Beschreibung

Die vorliegende Erfindung betrifft ein neues und verbessertes Verfahren zur Herstellung von 3,4-Epoxialkan- und 4-Hydroxi-2-alkensäuren und deren Ester durch Umsetzung mit 4-Hydroperoxi-2-alkensäuren oder deren Estern.

Aus der J. Polym. Sci., Polym. Chem. Ed., 22, 2501 (1984) sind Verfahren zur Herstellung der 3,4-Epoxialkansäuren und deren Ester bekannt, die auf der Epoxidierung mit m-Chlorperbenzoesäure basieren.

Sie sind wegen der hohen Kosten des Epoxidierungsmittels und der mehrstufigen Rückführung der als Abfallprodukte anfallenden m-Chlorbenzoesäure im technischen Maßstab unwirtschaftlich.

Aus der Tetrahedron Lett. 2417 (1973) und Tetrahedron 10, 4579 (1984) sind Verfahren zur Herstellung der 4-Hydroxi-2-alkensäuren und deren Ester bekannt.

Diese mehrstufigen Synthesen sind wegen des Preises und der Zugänglichkeit der verwendeten Reagentien nur schwer in einem technischen Maßstab zu übertragen.

Der vorliegenden Erfindung lag daher die Aufgabe zugrunde ein neues und verbessertes Verfahren zur Herstellung von 3,4-Epoxialkan- und 4-Hydroxi-2-alkensäuren und deren Ester zu finden und den zuvor genannten Nachteilen abzuhelfen.

Demgemäß wurden ein neues und verbessertes Verfahren zur Herstellung von 3,4-Epoxialkansäuren und deren Ester der allgemeinen Formel I 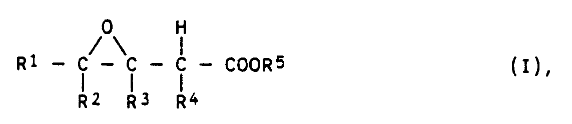
in der
- R¹, R², R³, R⁴, R⁵: Wasserstoff oder einen acyclischen, cyclischen, verzweigten oder unverzweigten Alkyl- oder Aralkylrest mit 1 bis 30 Kohlenstoffatomen bedeuten,
und 4-Hydroxi-2-alkensäuren und deren Ester der allgemeinen Formel II 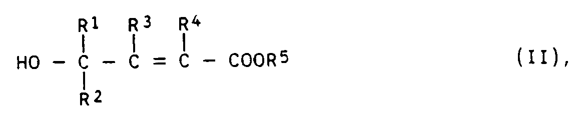
in der R¹ bis R⁵ die für Verbindungen I genannten Bedeutungen haben, gefunden, welches dadurch gekennzeichnet ist, daß man 3-Alkensäuren oder deren Ester der allgemeinen Formel III 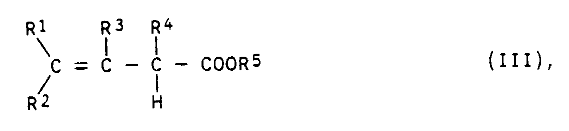
in der R¹ bis R⁵ die für die Verbindungen I genannten Bedeutungen haben, in Gegenwart eines Katalysators mit 4-Hydroperoxi-2-alkensäuren oder deren Ester der allgemeinen Formel IV 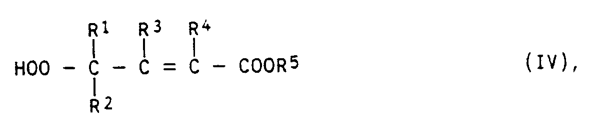
in der R¹ bis R⁵ die für die Verbindungen I genannten Bedeutungen haben, bei 50 bis 150°C und 0,01 bis 50 bar umsetzt.

Die 3,4-Epoxialkan- und 4-Hydroxi-2-alkensäuren und deren Ester und nach folgender Methode erhältlich:
Die Umsetzung erfolgt zwischen einer 3-Alkensäure oder deren Ester III und einer 4-Hydroperoxi-2-alkensäure oder deren Ester IV bei 50 bis 150°C und 0,01 bis 50 bar in Gegenwart eines geeigneten Katalysators nach folgender Reaktionsgleichung: 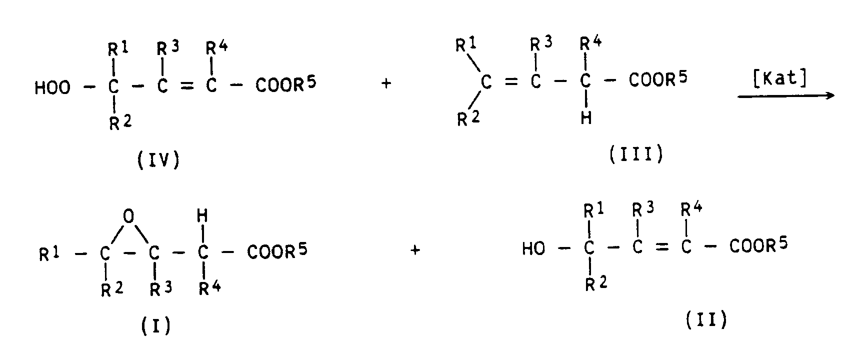
Die Reaktion kann kontinuierlich oder diskontinuierlich durchgeführt werden, wobei man die Verbindungen III und IV gemischt vorlegt und den Katalysator beifügt. Bevorzugt arbeitet man bei 50 bis 200°C und 0,1 bis 50 bar, besonders bevorzugt bei 90 bis 120°C und Atmosphärendruck.

Die Umsetzung kann in Gegenwart von Katalysatoren, vorteilhaft Übergangsmetallverbindungen der 4. bis 6. Gruppe des periodischen Systems, durchgeführt werden, z.B. von Verbindungen der Metalle Titan, Zirkon, Vanadium, Niob, Tantal, Chrom, Molybdän oder Wolfram. Geeignete Verbindungen sind beispielsweise Acetylacetonate oder Salze mit höheren Fettsäuren, z.B. mit 8 bis 18 Kohlenstoffatomen wie 2 Ethylhexansäure, Undecansäure, Stearinsäure oder Palmitinsäure, ferner Naphthenate. Besonders bevorzugt sind Verbindungen von Molybdän, Titan, Vanadium und Wolfram. Besondere Bedeutung haben Molybdän-Verbindungen erlangt. Vorteilhaft wendet man je Mol 4-Hydroperoxialkensäure oder deren Ester IV 0,1 bis 1,0 mmol der löslichen Katalysatoren, berechnet als Metall, an.

In aller Regel entstehen 3,4-Epoxysäuren I und 4-Hydroxysäuren II, zusammen im Verhältnis von 1:1.

Das eingesetzte Gemisch der Verbindungen III und IV kann auch ausgehend von 3-Alkensäuren oder deren Ester III durch Behandlung mit Sauerstoff haltigen Gasen bei 0 bis 150°C und 0,01 bis 50 bar, vorzugsweise bei 40 bis 120°C und 0,1 bis 5 bar, besonders bevorzugt bei 50 bis 100°C und Atmosphärendruck bis zu einem Umsatz von 10 bis 50 %, vorzugsweise 10 bis 20 % gewonnen werden und anschließend unter Zugabe eines Katalysators umgesetzt werden.

In den meisten Fällen genügen Verweilzeiten von 10 bis 120 Minuten, um Hydroperoxid-Umsätze von ≧90 % zu erzielen.

Nach den Umsetzungen wird der eingesetzte Katalysator entweder durch Filtration (bei Heterokatalysatoren) oder durch Extraktion (bei homogenen Katalysatoren) mit verdünnter Natronlauge entfernt. Das katalysatorfreie Reaktionsgemisch kann dann nach üblichen Methoden, z.B. fraktionierte Destillation, aufgearbeitet werden.

Die 3-Alkensäuren und deren Ester III sind allgemein bekannt oder können nach DE-A-2 630 086, Organic Synthesis, Coll. Vol. III, Seite 831 (1955), Tetrahedron Lett. 23, 3901 (1982) hergestellt werden.

Die Substituenten R¹, R², R³, R⁴ und R⁵ der 3,4-Epoxialkan- und 4-Hydroxi-2-alkensäuren und deren Ester haben unabhängig voneinander folgende Bedeutungen:
- Wasserstoff oder einen acyclischen, cyclischen, verzweigten oder unverzweigten Alkyl- oder Aralkylrest mit 1 bis 30 Kohlenstoffatomen, im speziellen
- Wasserstoff,
- unverzweigtes oder verzweigtes C₁-C₁₈-Alkyl, bevorzugt C₁-C₈-Alkyl, besonders bevorzugt C₁-C₈-Alkyl wie Methyl, Ethyl, n-Propyl, iso-Propyl, n-Butyl, iso-Butyl, sec.-Butyl und tert.-Butyl,
- C₃-C₁₂-Cycloalkyl, bevorzugt C₃-C₈-Cycloalkyl wie Cyclopropyl, Cyclobutyl, Cyclopentyl, Cyclohexyl, Cycloheptyl und Cyclooctyl, besonders bevorzugt Cyclopentyl und Cyclohexyl,
- C₄-C₁₈-Cycloalkyl-alkyl, bevorzugt C₄-C₁₂-Cycloalkyl-alkyl, besonders bevorzugt C₅-C₁₀-Cycloalkyl-alkyl wie Cyclohexyl-methyl, 1-Cyclohexyl-ethyl und 2-Cyclohexyl-ethyl
- C₄-C₁₈-Alkylycloalkyl, bevorzugt C₄-C₁₂-Alkylcycloalkyl, besonders bevorzugt C₅-C₁₀-Alkylcycloalkyl wie 2-Methylcyclopentyl, 3-Methylcyclopentyl, 2-Methylcyclohexyl, 3-Methylcyclohexyl, 4-Methylcyclohexyl und 2,4-Dimethylcyclohexyl,
- C₅-C₁₈-Alkylcycloalkyl-alkyl, bevorzugt C₅-C₁₂-Alkylcycloalkyl-alkyl, besonders bevorzugt C₆-C₁₀-Alkylcycloalkyl-alkyl wie 2-Methylcyclopentyl-methyl und 2-Methylcyclohexyl-methyl,
- C₇-C₁₈-Aralkyl, bevorzugt C₇-C₁₄-Aralkyl, besonders bevorzugt C₇-C₁₀-Aralkyl wie Benzyl, 1-Phenethyl und 2-Phenethyl.

Der Substituent R¹ bedeutet vorzugsweise Wasserstoff oder C₁-C₄-Alkyl, R² vorzugsweise Wasserstoff oder Methyl, R³ vorzugsweise Wasserstoff oder Methyl, R⁴ vorzugsweise Wasserstoff oder Methyl und R⁵ vorzugsweise Wasserstoff oder C₁-C₁₂-Alkyl, besonders bevorzugt C₁-C₈-Alkyl wie Methyl, Ethyl, n-Propyl, iso-Propyl, n-Butyl, iso-Butyl, sec.-Butyl, tert.-Butyl, Pentyl, Hexyl, Heptyl oder Octyl.

Bevorzugte 3,4-Epoxialkansäuren I und deren Ester sind:
3,4-Epoxi-buttersäure,
3,4-Epoxi-buttersäure-isobutylester,
3,4-Epoxi-2-methylbuttersäure,
3,4-Epoxi-2-methylbuttersäure-ethylester,
3,4-Epoxi-valeriansäure,
3,4-Epoxi-valeriansäure-methylester,
3,4-Epoxi-valeriansäure-isopropylester,
3,4-Epoxi-valeriansäure-hexylester,
3,4-Epoxi-valeriansäure-sek.-butylester.

Bevorzugte 4-Hydroperoxi-alken-säuren und deren Ester II sind:
Vinylessigsäure,
Vinylessigsäure-isobutylester,
2-Methyl-3-butensäure,
2-Methyl-3-butensäure-ethylester,
3-Pentensäure,
3-Pentensäure-methylester,
3-Pentensäure-isopropylester,
3-Pentensäure-n-hexylester,
3-Pentensäure-sek.-butylester.

Die 3,4-Epoxialkansäuren und deren Ester 1 sowie die 4-Hydroperoxi-2-alkensäuren und deren Ester II eignen sich als Zwischenprodukte zur Herstellung von 4-Ketosäuren und deren Ester, die als Weichmacher für Kunststoffe oder bei der Herstellung von pharmazeutischen Wirkstoffen Verwendung finden.

### Beispiele

### Beispiel 1

1856 g einer 7 %igen Lösung von 4-Hydroperoxi-2-pentensäuremethylester in 3-Pentensäuremethylester und 0,08 g Molybdän-ethylhexanoat wurden bei 100 bis 110°C in 9 h durch eine Kaskade von 3 Rührkolben (Gesamtvolumen 360 ml) gefahren. Der Hydroperoxid-Umsatz beträgt ca. 95 %. Der Austrag wird 2 x mit 50 ml 5 %iger Natronlauge sowie 2 x mit 50 ml Wasser gewaschen und im Rotationsverdampfer bei 40°C/25 mbar eingeengt.

Durch fraktionierte Destillation des Rückstands erhält man 87 g 3,4-Epoxi-valeriansäuremethylester mit dem Siedepunkt 34°C/8 mbar und 88 g 4-Hydroxi-2-pentencarbonsäuremethylester mit dem Siedepunkt 74°C/5 mbar.

### Beispiel 2

10 g Vinylessigsäure-i-butylester und 0,0015 g Molybdän-ethylhexanoat werden in einem Rührkolben vorgelegt, dazu bei 90°C unter Rühren 37 g einer 12 %igen Lösung von 4-Hydroperoxi-2-butensäure-i-butylester in Vinylessigsäure-i-butylester sowie 0,0015 g Molybdän-ethylhexanoat zugetropft und 1 h bei 90°C, danach noch 1 h bei 100°C gerührt. Der Hydroperoxid-Umsatz beträgt ca. 90 %.

Anschließend kühlt man ab auf RT und extrahiert mit 5 g 5 %iger wäßriger Natronlauge sowie 10 g Wasser. Die organische Phase enthält nach GC/MS 2,6 g Oxiranylessigsäure-i-butylester und 2,0 g 4-Hydroxi-2-butensäure-i-butylester.

### Beispiel 3

10 g 2-Methyl-3-butensäureethylester und 0,0012 g Molybdän-ethylhexanoat werden in einem Rührkolben vorgelegt, dazu bei 90°C unter Rühren 37 g einer 11 %igen Lösung von 4-Hydroperoxi-2-methyl-2-butensäureethylester in 2-Methyl-3-butensäureethylester sowie 0,0012 g Molybdän-ethylhexanoat zugetropft und 1 h bei 90°C, danach noch 1 h bei 100°C gerührt. Der Hydroperoxid-Umsatz beträgt ca. 95 %.

Anschließend kühlt man auf RT ab und extrahiert mit 5 g 5 %iger wäßriger Natronlauge sowie 10 g Wasser. Die organische Phase enthält nach GC/MS 1,0 g 3,4-Epoxi-2-methylbuttersäureethylester und 0,81 g 4-Hydroxi-2-methyl-2-butensäureethylester.

## Patentansprüche

1. Verfahren zur Herstellung von 3,4-Epoxialkansäuren und deren Ester der allgemeinen Formel I 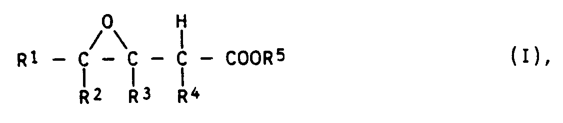 in der
R¹, R², R³, R⁴, R⁵ Wasserstoff oder einen acyclischen, cyclischen, verzweigten oder unverzweigten Alkyl- oder Aralkylrest mit 1 bis 30 Kohlenstoffatomen bedeuten,
und 4-Hydroxi-2-alkensäuren und deren Ester der allgemeinen Formel II 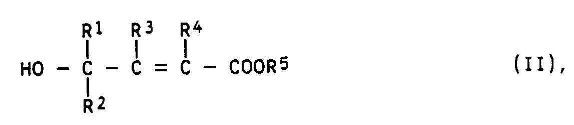 in der R¹ bis R⁵ die für Verbindungen I genannten Bedeutungen haben, dadurch gekennzeichnet, daß man 3-Alkensäuren oder deren Ester der allgemeinen Formel III 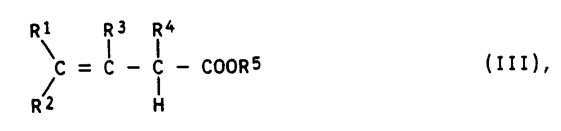 in der R¹ bis R⁵ die für die Verbindungen I genannten Bedeutungen haben, in Gegenwart eines Katalysators mit 4-Hydroperoxi-2-alkensäuren oder deren Ester der allgemeinen Formel IV 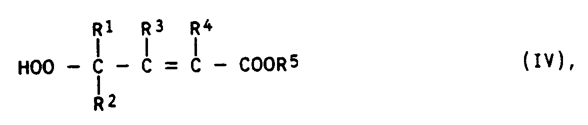 in der R¹ bis R⁵ die für die Verbindungen I genannten Bedeutungen haben, bei 50 bis 150°C und 0,01 bis 50 bar umsetzt.

2. Verfahren nach Anspruch 1, dadurch gekennzeichnet, daß die Substituenten in den Verbindungen I, II, III und IV
R¹, R², R³, R⁴, R⁵ Wasserstoff, C₁-C₂₀-Alkyl, C₃-C₁₂-Cycloalkyl, C₄-C₂₀-Cycloalkyl-alkyl, C₄-C₂₀-Alkylcycloalkyl, C₅-C₃₀-Alkylcycloalkyl-alkyl oder C₇-C₂₀-Aralkyl bedeuten.

3. Verfahren nach Anspruch 1, dadurch gekennzeichnet, daß man die 3-Alkensäuren oder deren Ester III mit sauerstoffhaltigen Gasen bei 20 bis 150°C und 0,01 bis 50 bar bis zu einem Umsatz von 10 bis 50 % behandelt und anschließend des vorliegende Gemisch aus den Verbindungen III und IV unter Zugabe eines geeigneten Katalysators umsetzt.

## Claims

1. A process for preparing 3,4-epoxyalkanoic acids and their esters of the general formula I 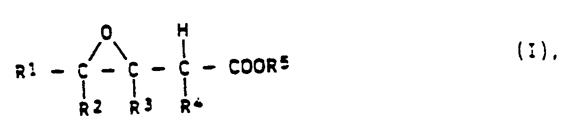 where
R¹, R², R³, R⁴, R⁵ are hydrogen or acyclic, cyclic, branched or unbranched alkyl or aralkyl with 1-30 carbon atoms,
and 4-hydroxy-2-alkanoic acids and their esters of the general formula II 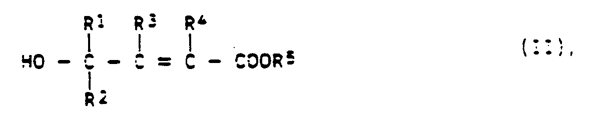 where R¹ to R⁵ have the meanings specified for compounds I, which comprises reacting 3-alkenoic acids or their esters of the general formula III 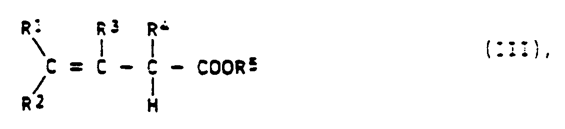 where R¹ to R⁵ have the meanings specified for compounds I, in the presence of a catalyst, with 4-hydroperoxy-2-alkenoic acids or their esters of the general formula IV 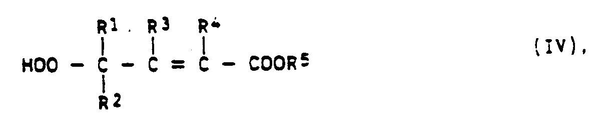 where R¹ to R⁵ have the meanings specified for compounds I, at 50-150°C under 0.01-50 bar.

2. A process as claimed in claim 1, wherein the substituents in compounds I, II, III and IV
R¹, R², R³, R⁴, R⁵ are hydrogen, C₁-C₂₀-alkyl, C₃-C₁₂-cycloalkyl, C₄-C₂₀-cycloalkylalkyl, C₄-C₂₀-alkylcycloalkyl, C₅-C₃₀-alkylcycloalkylalkyl or C₇-C₂₀-aralkyl.

3. A process as claimed in claim 1, wherein the 3-alkenoic acids of their esters III are treated with oxygen-containing gases at 20-150°C under 0.01-50 bar to a conversion of 10-50%, and then the resulting mixture of compounds III and IV is reacted with addition of a suitable catalyst.

## Revendications

1. Procédé de préparation d'acides 3,4-époxyalcanoïques et de leurs esters, de la formule générale I 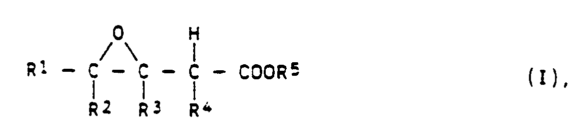 dans laquelle
R¹, R², R³, R⁴, R⁵ représentent chacun un atome d'hydrogène ou un radical aralkyle ou alkyle ramifié ou non ramifié, cyclique ou acyclique, lequel radical comporte de 1 à 30 atomes de carbone
et d'acides 4-hydroxy-2-alcénoïques et de leurs esters, de la formule générale II 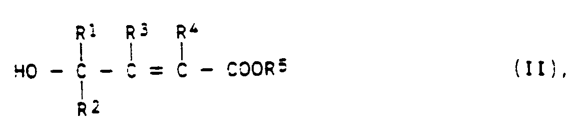 dans laquelle R¹ à R⁵ ont les significations qui leur ont été attribuées à propos de la définition des composés I, caractérisé en ce que l'on fait réagir des acides 3-alcénoïques ou leurs esters, de la formule générale III 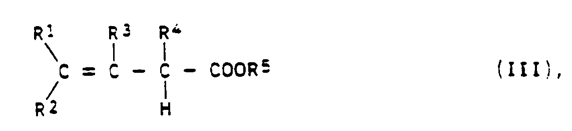 dans laquelle R¹ à R⁵ ont les significations qui leur ont été attribuées à propos de la définition des composés I, en présence d'un catalyseur, avec des acides 4-hydroxyperoxy-2-alcénoïques ou leurs esters, de la formule générale IV 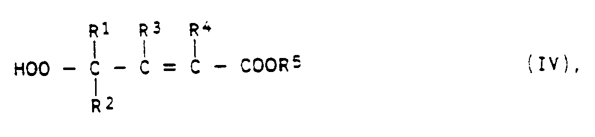 dans laquelle R¹ à R⁵ ont les significations qui leur ont été attribuées à propos de la définition des composés I, à des températures de 50 à 150°C et sous des pressions de 0,01 à 50 bars.

2. Procédé suivant la revendication 1, caractérisé en ce que dans les composés I, II, III et IV, les substituants
R¹, R², R³, R⁴, R⁵ représentent chacun un atome d'hydrogène, un radical alkyle en C₁ à C₂₀, cycloalkyle en C₃ à C₁₂, cycloalkyl-alkyle en C₄ à C₂₀, alkylcycloalkyle en C₄ à C₂₀, alkylcycloalkylalkyle en C₅ à C₃₀, ou aralkyle en C₇ à C₂₀.

3. Procédé suivant la revendication 1, caractérisé en ce que l'on traite les acides 3-alcénoïques ou leurs esters III par des gaz contenant de l'oxygène à 20-150°C et sous 0,01 à 50 bars, jusqu'à une conversion de 10 à 50% et on fait ensuite réagir le mélange obtenu des composés III et IV sous addition d'un catalyseur approprié.
